# EUROPEAN PATENT APPLICATION

(11) **EP 0 714 021 A1**
(43) Date of publication of application: **29.05.1996**
(21) Application number: 95610056.4
(22) Date of filing: 17.11.1995
(51) Int. Cl.: G01N 1/28, B01L 3/00, G01N 33/12

(54) **A sample container and a procedure to be used in the analysis of a sample**

(30) Priority: 24.11.1994 DK 1336/94
(71) Applicant: Slagteriernes Forskningsinstitut, DK-4000 Roskilde (DK)
(72) Inventor: Hagdrup, Claus, DK-2820 Gentofte (DK)

(57) **Abstract**

A sample container (1) to be used in the analysis of a food sample, e.g. a meat sample (11), comprises - as seen in its position of use - an upper section (2) which is open at the top to receive the sample, and in extension of this section a narrow lower section (3), which is closed at the bottom, for collection of liquid, e.g. meat juice (18) from the sample. When the liquid is to be analyzed, the upper section is removed and discarded, whereas the remaining lower section, which is designed with the same dimensions as a conventional test tube, is being used directly in an automatic analyzer. In this way the need for decanting of the secreted liquid from one container to another is eliminated.

## Description

This invention relates to a sample container to be used in the analysis of a sample, preferably a food sample.

It is generally known in the art that it may be injurious to your health to eat food products with too high a content of bacteria, e.g. salmonella.

Some products may also contain substances which in concentrations exceeding a certain level will give an unpleasant odour or taste to the product, which is unacceptable to the consumer. E.g. meat from male pigs may contain the substance skatole, which appears as an unpleasant odour when the meat is cooked.

In order to prevent such unacceptable products from entering the market, modern food plants keep a tight check on their production. This check takes place by regular taking of samples, which are analyzed to ascertain whether the prescribed standards are met.

Slaughterhouses take e.g. samples of meat, the juice of which is tested for bacteria. A sample of e.g. 10 g of meat is used. The sample is placed in a container having the shape of a test tube and the container is sealed and provided with a bar code label for identification of the sample. The sample is then frozen so that it can keep, e.g. during transportation.

Before the sample is analysed, it must be thawed. The meat juice secreted in the sample container is decanted into a smaller test tube, which may be placed in a rack along with a number of other tubes with meat juice from other samples. The rack is placed in an automatic analyzer, in which a pipette is successively entered down into tube after tube in order to collect a quantity of meat juice from each sample for analysis.

The mentioned decanting from the sample container to a test tube is necessary because the analyzer uses a pipette that requires a fairly high liquid column in order to operate reliably and effectively.

Such a liquid column cannot be formed to a satisfactory extent in the broad sample containers being used, which may e.g. have a diameter of 30 mm, whereas the test tubes normally will only have a diameter of 10 mm, and moreover, pipetting directly from the sample container may entail a considerable risk that the sample may accidentally clog up the pipette.

The work involved in decanting the meat juice from one container to another for each sample is troublesome and costly, considering the very high number of samples which are to be processed every day in a modern slaughterhouse.

In the test-tube-shaped sample containers the meat sample lies on the bottom of the container, resting with part of its surface on the wall of the container. As the meat juice is being secreted the sample will also - at any rate partly - be submerged in its own juice. Moreover, part of the meat juice will adhere to the sample container after the decanting. The result of this is that there will be relatively little meat juice available for the analysis.

Therefore, the purpose of the present invention is to provide a sample container of the type initially mentioned, which is easy and reliable to work with, which eliminates the need for decanting of the secreted liquid from one container to another, and which also allows collection of a larger quantity of meat juice than the known sample container.

This is achieved by the sample container of the invention, which is characterized in that the container - as seen in its position of use - has an upper section which is open at the top for receiving the sample, and in extension of this section a narrow lower section which is closed at the bottom for collection of liquid from the sample.

One advantage of the sample container of the invention is that the sample is not submerged in its own juice, as the secreted liquid is continuously drained from the upper section and immediately runs down into the lower section. Furthermore, only small quantities of liquid will remain in the upper section.

When the secreted liquid is to be analyzed the upper section of the container is just removed and discarded, whereas the remaining lower section of the container with the liquid can be used directly in an automatic analyzer, without any need for decanting. The lower section is preferably designed with the same dimensions as a conventional test tube. If desired, the upper section can be used again after the sample has been removed and the section has been washed.

In order effectively to drain liquid from the upper section, a funnel-shaped part with a central aperture may be provided between the two sections.

By forming a number of projections, e.g. in the shape of narrow ribs, on the inside of the upper section to carry the sample, by and large the entire surface of the sample is kept free allowing the liquid to secrete quickly and effectively.

The sample container may be made of plastic in one piece. When the secreted liquid in the lower section of the container is to be analyzed the container is cut through just below the upper section, which is then removed. The lower section may have an encircling weakening line near or at the transition to the upper section, so that the cutting through is facilitated, or the separation just means breaking off the upper section from the lower section.

In a preferred embodiment the two sections of the sample container are two separate parts which can be joined into one unit and detached from each other again. At first the unit serves as a sample container for receipt of the sample and secretion of liquid from this, and is then separated so that the lower section with the secreted liquid can be used as a test tube.

When the sample container consists of two detachable sections the upper section can also be provided with a tapering end and the lower section with a corresponding socket for easy and quick joining and detachment of the two sections.

The sample container of the invention can be provided with a lid to seal the upper section at the top when the sample has been put into the section. The lid or the upper section is preferably provided with a sealing lip in order to provide a tight joint when the lid has been put on the container.

When the container is made of plastic, the lid can advantageously be cast in one piece with the upper section of the container and attached to this by means of a foil hinge. In this way the lid is immediately at hand in an easy and convenient way when the container is to be closed.

The lid can suitably be provided with an encircling part that rests on the upper edge of the upper section, so that the lid can be opened fairly easily. The lid may have a projection which rests on the upper edge mentioned and protrudes from the periphery of the section, which makes the opening of the lid easier.

This is advantageous in particular when the upper section is to be reused, or if you wish to destruct samples and the plastic parts separately.

The invention also relates to a procedure to be used in the analysis of samples, preferably food samples. The procedure is characterized in,
- that a number of sample containers are provided, which - seen in their position of use - have an upper section which is open at the top, and in extension of this section a narrow lower section which is closed at the bottom,
- that a sample is placed in the upper section of each container,
- that the containers are placed in a rack,
- that the samples are brought to secrete liquid, which is collected in the lower section of each container,
- that the upper sections of the containers are removed,
- that the containers in the same or in another rack are placed in an analyzer, and
- that in the analyzer a quantity of liquid is taken from each lower section for analysis.

At a modern slaughterhouse a very high number of samples are being handled every day. These are therefore collected and handled suitably in racks which can hold e.g. 100-150 of the sample containers in question at the same time. The racks being used at the slaughter line are made of approved materials, such as plastic, steel or aluminium.

For use outside the slaughter line a rack made of e.g. carton can be used. The rack can consist of carton plates with holes of such a size that the lower section of the containers in question will fit these holes. Two racks complete with containers can be placed on top of each other in a carton box, the holes of the two racks being displaced in proportion to each other in such a way that the upper sections of the containers in the lower layer will be located between the lower sections of the containers in the upper layer. This arrangement will ensure that a given box can contain a maximum number of containers.

The box complete with an attached lid will form a practical packing for e.g. dispatch of a large number of samples to an external laboratory.

The method by which the samples are made to secrete liquid depends on the character of the samples.

When the sample is a piece of meat, the meat samples in the upper section of each of the sample containers can advantageously be made to secrete meat juice by first freezing the containers and then letting them stand for thawing the samples.

However, if the sample is a piece of fat, sample liquid can be made by heating the filled containers, so that the fat sample in the upper section of each container will melt out fat, which can be kept fluid by adding e.g. acetone.

When the sample itself is not likely to secrete liquid, the substances important for the analysis can be extracted by bringing the sample into contact with an extraction agent, e.g. by pouring a small quantity of extraction agent over the sample while the sample is in the upper section of the container.

The invention is described in further detail below, with reference to the drawings, wherein
- Fig. 1a-f illustrate a number of successive process steps with the use of a first embodiment of a sample container of the invention for analysis of a sample, the container being partly shown in section,
- Fig. 2 shows in detail the sample container used in the process steps of Fig. 1,
- Fig. 3 shows another embodiment of a container of the invention,
- Fig. 4 is a sectional side view of a fragment of a box with racks for a number of sample containers of the invention, and
- Fig. 5 is a top plane view of the same box with some of the parts removed in order better to illustrate the arrangement of the box.

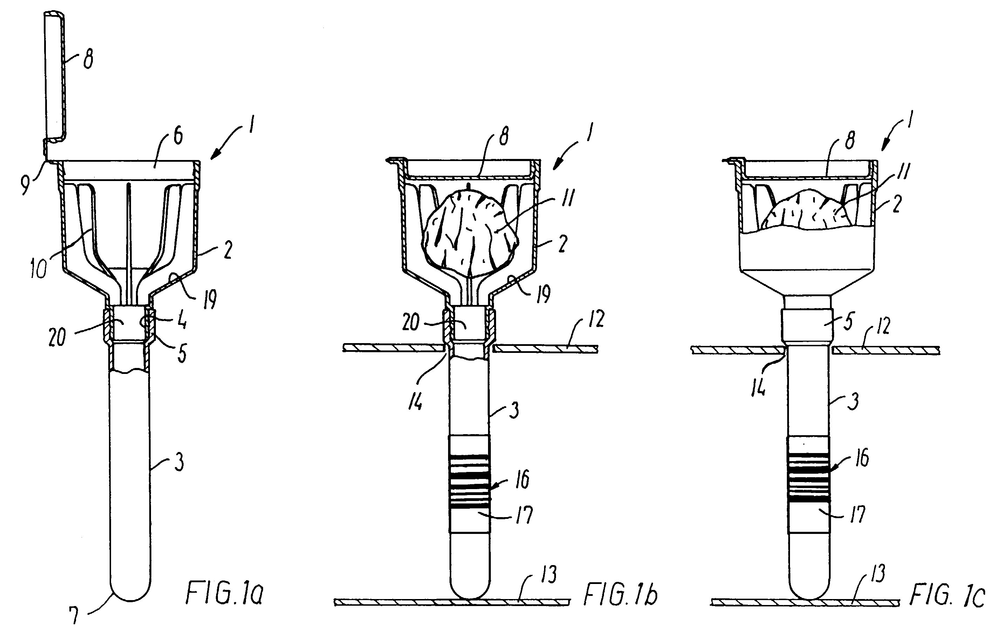

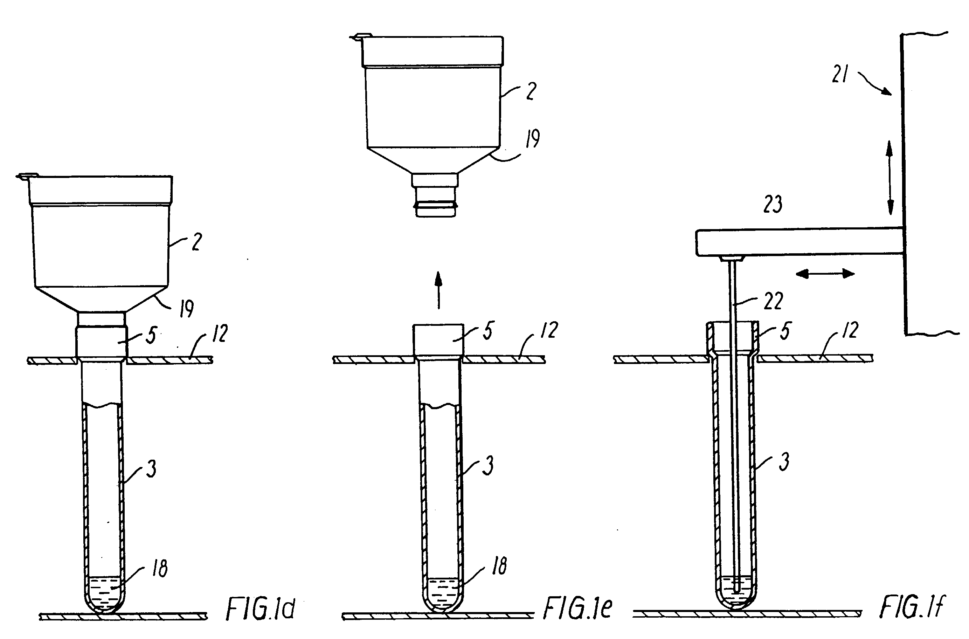

Fig. 1 a-f show a first embodiment of a sample container of the invention. The figures illustrate how the process is progressing successively using the sample container.

The sample container in its entirety has the reference number 1 and it is composed of an upper section 2 and a lower section 3. In this case, the two sections are separate parts which are joined into one unit by means of a tapering end 4 on the upper section 2 and a socket 5 on the lower section 3.

As illustrated, the upper section 2 is designed fairly wide with a free opening 6 at the top, whereas the lower section 3 is fairly narrow with a closed bottom 7 below.

By the shown embodiment the sample container 1 is made of plastic, and it has a lid 8, which is fastened to the upper section 2 by means of a foil hinge 9. The lid serves to seal the opening 6 of the upper section after the sample has been put in.

The inside of the upper section 2 is provided with cast ribs 10, which are placed at identical intervals along the periphery. The ribs 10 serve to support the sample so that the greater part of its surface can freely secrete liquid.

The sample container of the invention can be used for many different types of samples, e.g. of food, which are to be checked for any unwanted bacteria or accompanying substances, and which are preferably able to secrete a liquid into the sample container.

The process is described below with a meat sample taken from a carcass.

In Fig. 1a the two sections 2 and 3 of the container 1 are joined into one unit by means of the tapering end 4 of the upper section 2 and the socket 5 of the lower section 3. The lid 8 is open and the container is ready to receive a meat sample 11.

In Fig. 1b the meat sample 11 has been put into the upper section 2 of the container, which has then been closed by the lid 8. A label 16 with a bar code 17 has been stuck on the lower section of the container. The bar code serves to later identification of the sample in question and its origin.

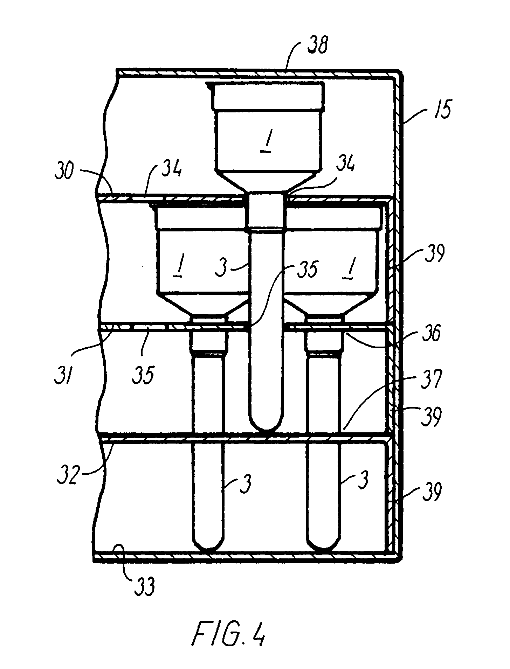

Furthermore, the container is placed in a rack, here only indicated fragmentarily, which comprises an upper plate 12 and a lower plate 13. The upper plate 12 is provided with a hole 14 to receive the lower section 3, the bottom 7 of which rests on the lower plate 13. Fig. 1b-f show only part of the rack with a single container, whereas a complete rack is designed to hold a large number of containers. Moreover, two racks complete with containers, as shown in Fig. 4, can be placed on top of each other and placed in a common box 15.

The box 15 complete with filled sample containers placed in their racks is frozen (Fig. 1c). In this way the samples can be stored in almost unchanged condition until they later are to be analyzed in an internal or external laboratory. In the latter case the box with samples may be placed in an insulating box to prevent the samples from being thawed at the wrong moment during transportation or during storage at the laboratory.

In Fig. 1d the samples have reached the thawing stage. During thawing the meat secretes some meat juice 18, which runs from the upper section 2 down into the lower section 3 below. As the sample only rests on the narrow ribs 10, by and large the entire surface of the sample can secrete meat juice.

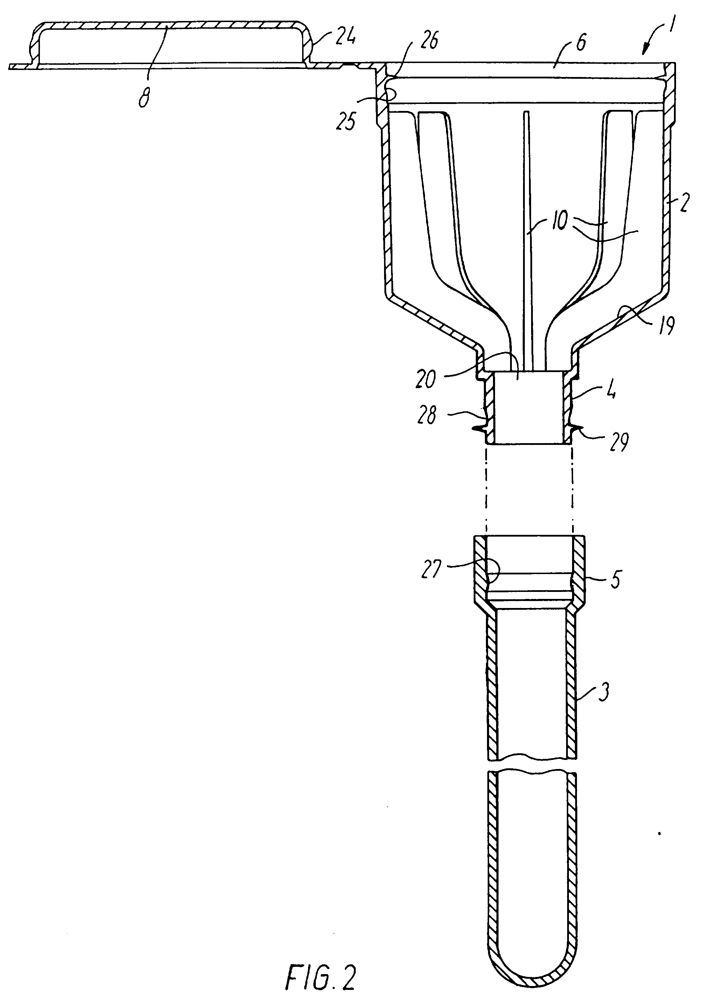

The meat juice will either drip directly down into the lower section 3 below or upon the bottom of the upper section, which is therefore shaped like a funnel 19 with a central aperture 20 (Fig. 2). The funnel shape ensures that the meat juice dripping down will quickly proceed down into the section 3 below via the central aperture 20, and that only small quantities of meat juice will remain on the bottom of the upper section.

In Fig. 1e the two sections 2 and 3 are detached and the upper section 2 with the meat sample 11 is thrown away, whereas the lower section 3 with the secreted meat juice 18 will remain in the plates 12, 13 of the rack. The remaining part of the sample container 1, i.e. the lower section 3, now passes on in the process as a test tube, and therefore it has the same dimensions as a conventional test tube.

Depending upon the arrangement of racks and analyzer, the filled test tube can be introduced into the analyzer in the same rack or after having been transferred to another rack.

In Fig. 1f the filled test tube is now finally in the automatic analyzer 21, which is only shown in schematic outline. Along with the shown test tube the apparatus will normally contain a number of tubes with meat juice which is to be sucked up for analysis.

This sucking up takes place by means of a pipette 22, located on a robotic arm 23, which successively lowers the pipette into one tube after the other in order to suck up a quantity of liquid for analysis of the meat juice from the meat samples in question, which, as mentioned before, can be identified by means of the bar code 17 on the label 16.

The functioning of the pipette requires an adequately high liquid column, which is formed in the container being used here, because the lower section is as narrow as conventional test tubes.

Prior to the mentioned pipetting, other procedures can be performed, if desired, such as admixture of diluent and/or reagents, or centrifuging.

In Fig. 2 the design of the first embodiment of the sample container is seen in larger scale and in more detail than in Fig. 1a-f. The lid 8 is provided with a encircling bead 24 and on the inside of the upper section 2 is a corresponding encircling groove 25. When the lid is closed over the put-in sample, the bead and the groove will interlock and retain the lid in a closed position. To seal the joint between the lid and the upper section the inside of the section is provided with an encircling sealing lip 26.

In order to keep the upper section 2 and the lower section 3 sufficiently well together, yet detachable, when they are joined and are to function as a unit, an encircling bead 27 is formed on the inside of the socket 5 and the outside of the tapering end 4 has a corresponding encircling groove 28. An encircling sealing lip 29, designed on the outside of the tapering end, serves to seal the joint between the socket and the tapering end when the sections are joined.

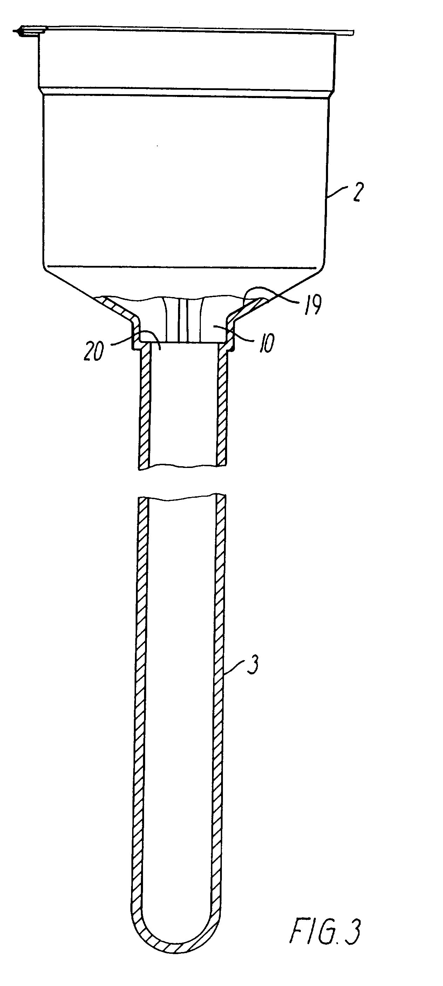

Fig. 3 shows fragmentarily another embodiment of a sample container of the invention. This container is made in one piece, but in other respects it corresponds to the first embodiment as shown in Fig. 1 and 2, and for identical parts the same reference numbers are therefore used.

In use the container goes through process steps that correspond to the ones shown in Fig. 1a-d and Fig. f. However, the container, which now consists of only one piece, cannot be dismanteled as shown in Fig. 1e. Instead, the upper section is cut off in a way not illustrated and then discarded.

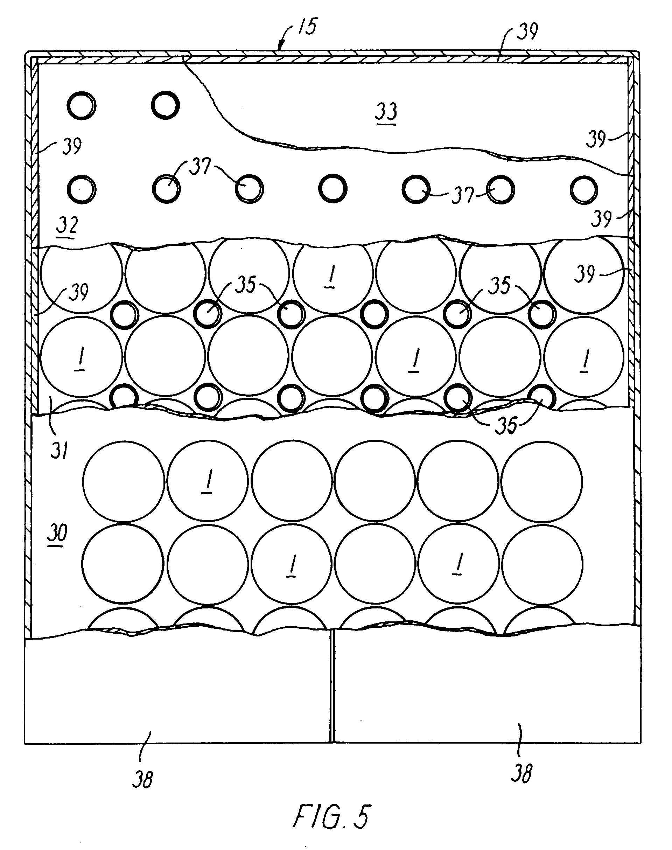

Fig. 4 and 5 show in detail the arrangement of the racks and the box to match.

The racks are designed with plates 30, 31, 32, and 33, which can be made of carton or another suitable material, e.g. plastic, and together they constitute two racks.

The first rack consists of the plates 30, 31 and 32, and the other one of the plates 31, 32 and 33. As is seen, the plates 31 and 32 are common for the two racks, and the two racks are dislocated vertically in proportion to each other by a distance which substantially corresponds to the height of the upper section of a container. The individual plates are furthermore kept at a distance from each other and support each other by means of spacers 39, which consist of flaps 39, which are bent 90° downwards on each of the three plates 30, 31 and 32.

The plates 30, 31 of the first rack are provided with flushing holes 34 and 35 to receive the lower narrow section 3 of a container, which also rests its bottom 7 on the lower plate 32 of the rack, which plate is also the middle plate in the other rack. The upper section 2 of the container protrudes over the top plate 30 of the rack.

In the two top plates 32, 33 of the other rack there are corresponding flushing holes 36 and 37 to receive the lower narrow section 3 of a container, which also rests its bottom 7 on the lower plate 33 of the rack, which plate also serves as the bottom of the box 15. The upper section 2 of the container protrudes over the top plate 31 of the rack and into the space between the lower sections 2 of the upper layer of containers.

The holes of the two racks, 34, 35 and 36, 37 respectively, are therefore displaced horizontally in proportion to each other in such a way that one set of holes in one of the racks lies in the middle between four sets of holes in the other rack. The distance from the center of a hole in one of the racks to each of the centers of the adjecent four holes in the other rack corresponds to half the sum of the diameters of the two sections of a container, or slightly bigger than this sum.

The holes in the racks can also be located so that the containers in the upper rack will be placed closed to the sides of the box, whereas the containers in the lower rack will be placed at some distance from the sides of the box, i.e. the opposite of what is illustrated in Fig. 4 and 5.

The racks can also be made of foamed plastic, e.g. the so-called "flamingo", by which the upper rack can have the shape of a plate of a thickness corresponding to the space between the plates 30 and 31, such plate being provided with holes for the upper sections and the lower sections of the containers at this level. The lower rack can consist of a plate of a thickness which corresponds to the space between the plates 31 and 33, the plate having holes for the lower sections of the containers at this level, so that the containers will for example stand to form the pattern shown in Fig. 4. After having been filled with containers the foamed plates can be placed in a box corresponding to the box 15. An advantage of using racks of foamed plastic is that they can be used on the slaughter line, so that the containers can be put directly into a rack after they have been provided with a meat sample.

With the above-mentioned rack structures it is possible advantageously to store the sample containers so that they take up as little room as possible.

The containers and racks in question can for example be used in the examinations mentioned initially, i.e. in the examination of meat for salmonella bacteria, or fat for the content of skatole, but they can also be used in the examination of any other samples, e.g. in the examination of meat, fat or other animal tissue for other substances.

In Fig. 1b a single sample container with a sample has just been placed in one rack, in this case only indicated by plates 12, 13. However, the rack design described above is intended for a large number of sample containers and it is used in the following way.

At first the lower rack 31, 32 and 33 of the box 15 is filled with sample containers, while the top plate 30 is removed. Next, this plate 30 is positioned in the box, and the upper rack 30, 31 and 32 is filled with sample containers, and then the lid 38 is closed on the filled box, which can e.g. be frozen, if it contains meat samples, and/or serve as transport packaging.

## Claims

1. Sample container (1) to be used in the analysis of a sample (11), **characterized** in that the container - as seen in its position of use - has an upper section (2) which is open at the top for receipt of the sample, and in extension of this section a narrow lower section (3) which is closed at the bottom for collection of liquid (18) from the sample.

2. Sample container according to Claim 1, **characterized** in that the upper section (2) extends into the lower section (3) via a funnel-shaped part (19) having a central aperture (20).

3. Sample container according to Claim 1 or 2, **characterized** in that the inside of the upper section (2) is provided with a number of spaced projections (10).

4. Sample container according to Claim 1, 2, or 3, **characterized** in that the two sections (2, 3) of the container are separate parts which have been designed to be joined with each other in a detachable way.

5. Sample container according to Claim 4, **characterized** in that the two separate sections (2, 3) of the container are provided with a tapering end (4) and a socket (5), respectively, for detachable joining of the two sections with each other, and that the tapering end or the socket is preferably provided with a sealing lip (29) to seal the joint when the sections have been joined.

6. Sample container according to Claim 5, **characterized** in that the socket (5) and the tapering end (4) are provided with a encircling bead (27) and an encircling groove (28), which will interlock when the two sections are joined.

7. Sample container according to one of Claims 1 - 6, **characterized** in that the container (1) is provided with a lid (8) to close in a detachable way the upper section (2) at the top, and that the lid or the upper section is preferably provided with a sealing lip (26) to seal the joint when the lid has been put on the container.

8. Sample container according to one of Claims 1 - 7, **characterized** in that the container (1) is made of plastic and that the lid (8) is attached to the upper section (2) by means of a foil hinge (9).

9. Procedure to be used in the analysis of samples, **characterized** in,
- that a number of sample containers are provided, which - seen in their position of use - have an upper section which is open at the top, and in extension of this section a narrow lower section which is closed at the bottom,
- that a sample is placed in the upper section of each container,
- that the containers are placed in a rack,
- that the samples are brought to secrete liquid, which is collected in the lower section of each container,
- that the upper sections of the containers are removed,
- that the containers in the same or in another rack are placed in an analyzer, and
- that a quantity of liquid is taken in the analyzer from each lower section for analysis.

10. Procedure according to Claim 9, to be used on a meat sample, **characterized** in that a meat sample in the upper section is brought to secrete meat juice, which runs down into the lower section, by first freezing the filled containers placed in a rack, and then storing them at a temperature higher than 0 °C for such a period of time that the meat pieces have at least been partly thawed.

11. Procedure according to Claim 9, to be used on a fat sample, **characterized** in that a fat sample in the upper section is brought to melt out fat, which runs down into the lower section, by heating the filled containers placed in a rack.

12. Procedure according to Claim 9, **characterized** in that the sample in the upper section of a container is brought into contact with an extraction agent, which in the form of an extract runs down into the lower section.
